# EUROPEAN PATENT SPECIFICATION

(11) **EP 1 671 674 B1**
(45) Date of publication and mention of the grant of the patent: **17.10.2007**
(21) Application number: 05027054.5
(22) Date of filing: 10.12.2005
(51) Int. Cl.: A61Q 5/00, A61Q 5/06, A61K 8/81

(54) **Use of fluorescent polymers for the treatment of human hair**
Verwendung von fluoreszierenden Polymeren für die Behandlung menschlichen Haares
Utilisation des polmères fluorescents pour le traitment des cheveux humaines

(30) Priority: 14.12.2004 DE 102004060033
(43) Date of publication of application: 21.06.2006
(73) Proprietor: Wella Aktiengesellschaft, 64274 Darmstadt (DE)
(72) Inventor: Speckbacher, Markus, 3175 Flamatt (CH); Baumeister, Jan, 1726 Farvagny-le-Grand (CH); Krause, Thomas, 64297 Darmstadt (DE)

(56) References cited:
- EP-A- 0 337 951
- EP-A- 0 422 535
- DE-A1- 3 814 647
- US-B1- 6 391 062

## Description

Object of the invention is the use of fluorescent polymers for the treatment of human hair, especially for the improvement of hair gloss, as well as the corresponding hair treatment agents, fluorescent polymers and polymerizable fluorescent monomers for their preparation.

In the hair cosmetic market, especially the hair styling market, the demand for products with new properties is still very large. Product innovations that appeal to several senses concomitantly, arouse positive associations or differ from conventional products by reason of exceptional additional properties have high potential to command attention. For example, hair cosmetics that shine strongly under black light (e.g. in the disco) and/or are able to enhance the gloss of the hair or the brilliance of the hair color in daylight and/or are able to serve as indicators for diagnosis of the state of the human hair through a content of suitable substances are of interest. In principle, fluorescent dyes would be suitable for this purpose. However, many known fluorescent dyes are not totally satisfactory in their actions since, for example, the effect is not adequate or the cosmetic compatibility or the compatibility with product contents is not entirely satisfactory. The gloss of human hair and the brilliance of hair colors is of considerable importance in hair cosmetics. Shining hair is usually associated with healthy, natural, vital hair. There is a whole series of products for improving the sheen of hair. Many products based, for example, on oils or silicone compounds do indeed enhance the gloss of the hair, but at the same time have detrimental side effects. In particular, they can stress the hair, which complicates the use on fine hair. There is therefore a demand for further products that enhance hair gloss, color effects on the hair, or the brilliance of hair colors.

It has now been found that fluorescent polymers are highly suitable for the treatment of human hair, especially for the production of gloss and fluorescent color effects. Object of the invention is therefore the use of fluorescent polymers for the cosmetic treatment of human hair, especially the use for the enhancement of gloss or brilliance of human hair. In the preparation of the polymers homo- and copolymers may be obtained by inclusion polymerization of functionalized fluorescent dyes. The fluorescent polymers can also act as thickeners, compatibility enhancers or as film-forming styling polymers when used as hair cosmetics. Depending upon the monomer composition, they are characterized by a specific absorptive behavior on the hair. Through their fluorescence, the absorptive behavior of these polymers can be used as indicators for certain hair conditions, for example hair damage.

Fluorescent compounds are substances that are able to absorb light of one wave length that lies preferably in the UV range (< 400 nm), and to radiate light of a higher wave length that lies preferably in the visible range (> 400 nm).

Perylene dyes have been known for a long time as highly lightfast and stable fluorescent dyes with very high fluorescent quantum yields (e. g. WO 00/23446; DE 19651712). They are used therefore in different applications where excellent fluorescence properties are required. A method is described in DE 10233179 A1 that enables the preparation of certain fluorescent polymers, in which the dye molecules are bonded covalently with the polymer. The method for efficient inclusive polymerization of polymerizable fluorescent dyes into a selected support polymer described therein allows the preparation of highy lightfast and solvent resistant fluorescent polymers.
EP 422 535 A1 discloses polymerisable perylene-3,4,9,10-tetracarboxylic diimides. According to EP 422 535 it has been an objective to obtain monomers which are easy to polymerize in that polymerization is not inhibited by steric effects. Also, the polymerized dyes should be stable vs. temperature and light, show a high dichroism and -upon use as components in liquid-cristalline polymer- should modify the liquid-cristalline-matrix as little as possible.
EP 337 951 A2 refers to colored polymer microparticles having a particle size of between 0.01 and 0.5 µm, which are obtainable by conventional copolymerization of one or more different mono- or polyethylenically unsaturated compounds and/or one or more glycidyl compounds and/or one or more different monomers selected from the group comprising polyalcohols, polycarboxylic acids, hydroxycarboxylic acids, lactones and aminocarboxylic acids. In addition, at least one dye or pigment derivative which contains a reactive group capable of polymerization is copolymerized.
DE 3814647 A1discloses perylene-3,4,9,10-tetracarboxylic diimideshaving a dielectric coefficient of up to 110. The dyes are said to have a high thermal, chemical, photochemical and radiation-chemical stability, whereby they can also be used under adverse operating conditions.
U.S. 6,391,062 B1 relates to the use of fused polycyclic compounds having at least one cationic group Z, Z being chosen from quaternized aliphatic chains, aliphatic chains having at least one quatemized saturated ring and aliphatic chains having at least one quaternized unsaturated ring, as direct dyes in compositions intended for dyeing keratin substances. In particular, the compositions are intended for dyeing human keratin fibers, especially the hair, and cosmetic compositions intended for making up the skin, the nails and the lips. According to the invention not only these but also other polymerizable fluorescent dyes may be homopolymerized or copolymerized with one or more further non-fluorescent monomers, and by application to human hair novel effects may be achieved with the fluorescent polymers thus obtained

### Polymerizable, fluorescent monomers

Preferred fluorescent polymers are constructed of at least one type of fluorescent monomer that has at least one perylene or at least one naphthalene bislactam structural unit. These monomers are preferably unsaturated ethylenic, radically polymerizable monomers. The perylene structural units are preferentially tetracarboxylic acid bisimides or hexacarboxylic acid trisimides, in which at least one of the nitrogen atoms is substituted with a suitable vinyl group. In the case of the naphthalene bislactams too the polymerizable vinyl group is present preferably as a substituent on a nitrogen atom.

Preferred radically polymerizable unsaturated ethylenic fluorescent monomers are selected from monomers of the formula 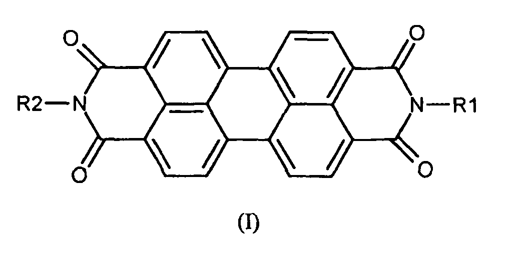 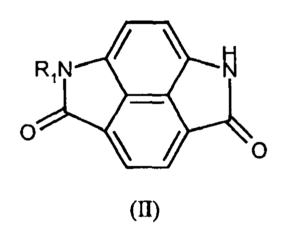 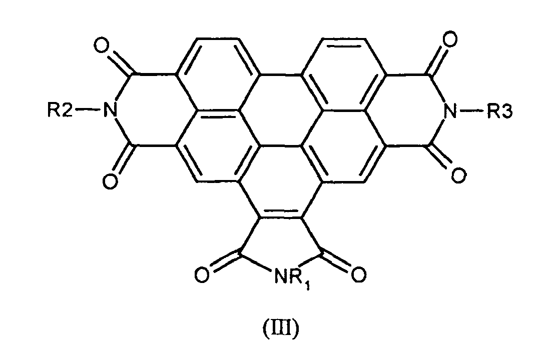 in which R1 stands for an unsaturated ethylenic group and R2 and R3 independently from one another stand for hydrogen or an organic residue with 1 to 24 C atoms, especially a linear or branched C1-24 alkyl group, or for a residue of the formula (IV) or (V).

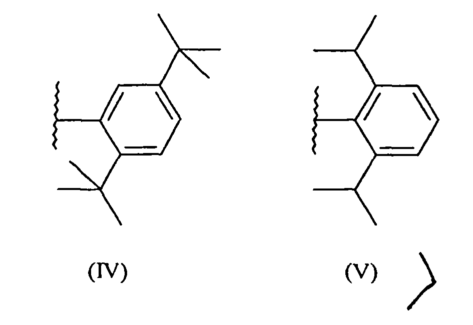

The residue R1 has preferably the general formula -X-CR=CH2, in which X stands for a C1 to C18 alkylene group or for a phenylene group, preferably for a linear C1 to C18 alkylene chain such as methylene, ethylene or propylene or preferably for a 1,2-, 1,3- or 1,4-phenylene group.

Polymerizable, fluorescent monomer dyes of the general formula (I) and the synthesis of the corresponding polymers are described in DE 102 33 179 A1.

Object of the invention are dyes of the general formula (II). They belong to the class of naphthalene bislactams. The basic structure is known and is accessible preparatively according to the work of S. S. Dalvi et. al. (Indian Journal of Chemistry, Vol. 24B, April 1985, 377-382). These compounds are colored differently according to the substitution pattern, e.g. red, pink or blue-violet. According to the invention naphthalene bislactams with vinyl substituents can be prepared as follows. 6-Bromo-2-oxo-1,2-dihydrobenzo[cd]indole-5-carboxylic acid (a) (also known as 6-bromo-5-naphthostyril carboxylic acid) can be prepare in a multi-stage synthesis starting from naphthalene-1,4- dicarboxylic acid (Indian Journal of Chemistry, vol. 24B, April 1985, 377-382). The vinyl-substituted bislactams according to the invention (c) are accessible by the Ullmann condensation of aromatic amines (e.g. 4-vinylaniline (b)) and 6-bromo-2-oxo-1,2-dihydrobenzo[*cd*]indole-5-carboxylic acid (a).

For example, the synthesis diagram for 1-(4-vinylphenyl)-1,6-dihydroisoindolo[6,7,1-*cde*]indole-2,5-dione (c):

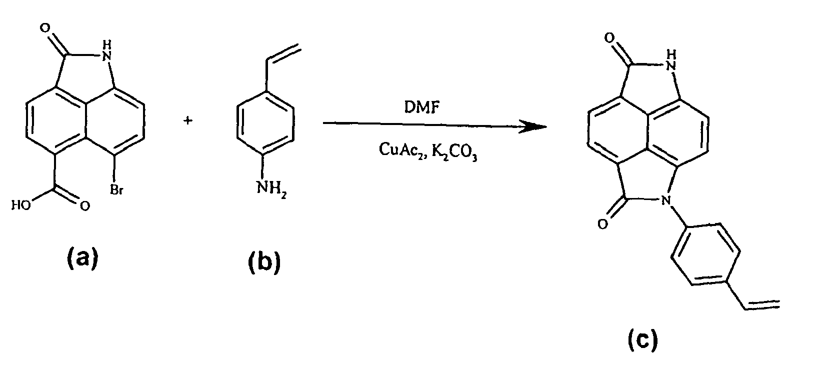

Object of the invention are also dyes of the general formula (III). They belong to the class of the perylene hexacarboxylic acid trisimides. As example the following synthesis is described. By nucleophilic substitution of N',N'bis(I-hexylheptyl)-benzo[ghi]perylene-2,3,8,9,11,12-hexacarboxylic acid 2,3:8,9:11,12-tris(dicarboximide) (**d**) (synthesis described in Eur. J. Org. Chem. 2001, 2481-2486 and WO 00/23446) with allyl bromide (**e**) in DMF and potassium carbonate as base N¹-(1-propen-3-yl)-[N²,N³-bis(1-hexylheptyl)-benzo[ghi]perylene-2,3,8,9,11,12-hexacarboxylic acid 2,3:8,9:11,12-tris(dicarboximide) (**f**) as derivative of the general formula (III) according to the invention is obtained. Synthesis diagram:

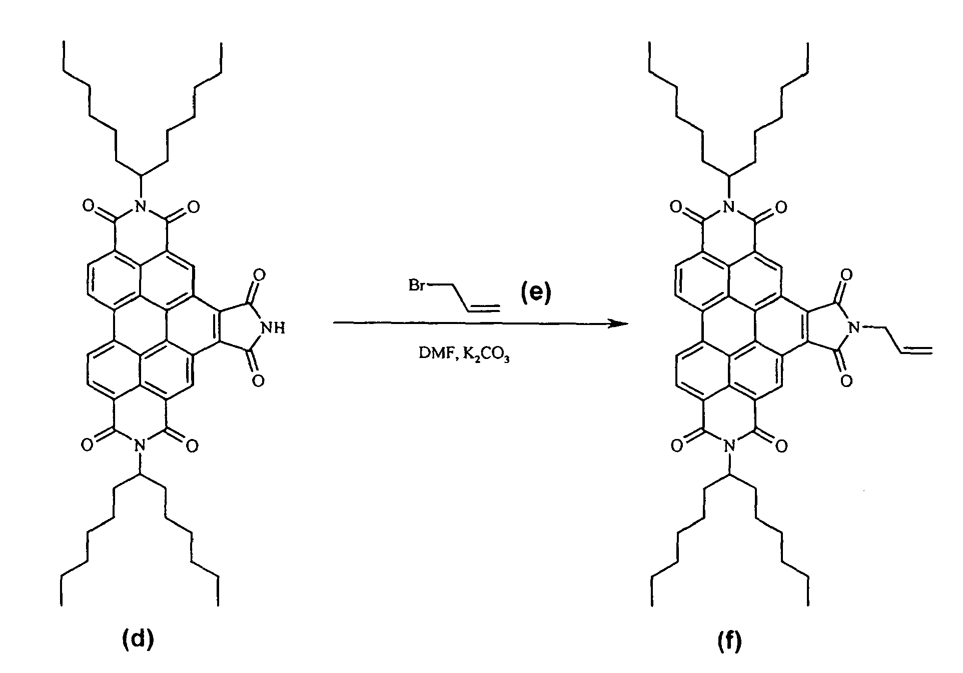

Object of the invention are also fluorescent polymers that are constructed of at least one type of fluorescent monomer that has at least one naphthalene bislactam structure unit or at least one perylene hexacarboxylic acid trisimide structure unit, especially from fluorescent monomers of the formula (II) and (III), e.g. from 1-alkenyl-1,6-dihydroisoindolo[6,7,1-*cde*]indole-2,5-diones or from N'-alkenyl-[N²,N³-bis(alkyl)-benzo[ghi]perylene-2,3,8,9,11, 12-hexacarboxylic acid 2,3:8,9:11,12-tris(dicarboximide).

### Fluorescent polymers

Suitable fluorescent polymers for hair cosmetic use that may be prepared by homo- or copolymerization of the aforementioned fluorescent monomers include non-ionic, anionic, cationic, zwitterionic or amphoteric polymers. Preferred fluorescent polymers are constructed from at least one type of fluorescent monomer that contains at least one naphthalene bislactam structural unit or at least one perylene hexacarboxylic acid trisimide structure unit especially from monomers of the aforementioned formulae (II) and (III), e.g. 1-alkenyl-1,6-dihydroisoindolo[6,7,1-*cde*]indole-2,5-dione and N¹-alkenyl-[N²,N³-bis(alkyl)-benzo[ghi]perylene 2,3,8,9,11, 12-hexacarboxylic acid-2,3:8,9:11,12 tris(dicarboximides), whereby the alkyl groups can have, for example, 1 to 30 C atoms and the alkenyl group has preferably 3 to 30 C atoms, e.g. 1-propen-3-yl or 1-(4-vinyl)phenyl.

Non-ionic copolymers are formed, for example, by copolymerization of the unsaturated ethylenic fluorescent monomers with acrylamide, methacrylamide, alkyl- and dialkylacrylamide, alkyl- and dialkylmethacrylamide, alkyl acrylate, alkyl methacrylate, vinylcaprolactone, vinylcaprolactam, vinylpyrrolidone, vinyl alcohol, vinyl ethers, vinyl esters, e.g. vinyl acetate, whereby the alkyl groups of these monomers are preferably C1 to C7 alkyl groups, especially preferred C1 to C3 alkyl groups. Also suitable are styrene and its non-ionic derivatives, alkyl- or aryl-substituted cyclic maleimides, such as, for example, N-phenylmaleimide.

Suitable amino-substituted vinyl monomers are, for example, aminoalkyl acrylate, aminoalkyl methacrylate, dialkylaminoalkyl acrylate, dialkylaminoalkyl methacrylate, monoalkylaminoalkyl acrylate and monoalkylaminoalkyl methacrylate, whereby the alkyl groups of these monomers are preferably C1 to C7 alkyl groups, especially preferred C1 to C3 alkyl groups. Also 2 methacryloyloxyethylphosphoryl choline and its derivates can be copolymerized.

Anionic copolymers can be obtained by polymerization with monomer units containing acid groups, whereby optionally further comonomers that contain no acid groups present can be copolymerized. Suitable anionic comolymers are unsaturated ethylenic carboxylic acids sulfonic acids or phosphoric acids, especially acrylic acid or methacrylic acid, crotonic acid, maleic acid or maleic anhydride, maleic acid monoesters, especially the mono- C1-C7 alkyl esters of maleic acid, itaconic acid, styrene sulfonates, amidoalkylacrylamide sulfonates as well as aldehydocarboxylic acids or ketocarboxylic acids.

Preferred anionic fluorescent copolymers are copolymers of the aforementioned fluorescent monomers with the following additional non-fluorescent monomers: copolymers with acrylic acid, alkyl acrylate and N-alkylacrylamide, especially copolymers with acrylic acid, ethyl acrylate und N-tert-butylacrylamide; cross-linked or uncrosslinked copolymers with vinyl acetate and crotonic acid; copolymers with one or more C1-C5 alkyl acrylates, especially C2-C4 alkyl acrylates and at least one monomer selected from acrylic acid or methacrylic acid e.g. copolymers with tert-butyl acrylate, ethyl acrylate and methacrylic acid; copolymers with sodium polystyrene sulfonate; copolymers with vinyl acetate, crotonic acid and vinyl alkanoate, e.g. copolymers with vinyl acetate, crotonic acid and vinyl propionate; copolymers with vinyl acetate, crotonic acid and vinyl neodecanoate; copolymers with aminomethylpropanol acrylate; copolymers from vinylpyrrolidone and at least one further monomer selected from acrylic acid and methacrylic acid as well as optionally acrylic acid esters and methacrylic acid esters; copolymers with methyl vinyl ether and monoalkyl maleates; aminomethylpropanol salts of copolymers with allyl methacrylate and at least one further monomer selected from acryl acid and methacrylic acid as well as optionally acrylic acid esters and methacrylic acid esters; cross-linked copolymers with ethyl acrylate and methacrylic acid; copolymers with vinyl acetate, mono-n-butyl maleate and isobornyl acrylate; copolymers with two or more monomers selected from acrylic acid and methacrylic acid as well as optionally acrylic acid esters and methacrylic acid esters; copolymers with octylacrylamide and at least one monomer selected from acrylic acid and methacrylic acid as well as optionally acrylic acid esters and methacrylic acid esters, whereby the alkyl groups of the aforementioned polymers have usually preferably 1, 2, 3 or 4 C atoms.

Zwitterionic or amphoteric copolymers with fluorescent monomers are, for example, copolymers formed by copolymerization with alkylacrylamide, alkylaminoalkyl methacrylate and two or more monomers from acrylic acid and methacrylic acid as well as optionally their esters, especially copolymers from octylacrylamide, acrylic acid, butylaminoethyl methacrylate, methyl methacrylate and hydroxypropyl methacrylate; copolymers with at least one type of monomer that contains quaternary amino groups and at least one further type of monomer that contains acid groups; copolymers with fatty alcohol acrylates, alkylamine oxide methacrylate and at least one monomer selected from acrylic acid and methacrylic acid as well as optionally acrylic acid esters and methacrylic acid esters, especially copolymers with lauryl acrylate, stearyl acrylate, ethylamine oxide methacrylate and at least one monomer selected from acrylic acid and methacrylic acid as well as optionally their esters; copolymers with betaines, e.g. methacryloylalkyl betaine, quaternary croton betaines or quaternary croton betaine esters; copolymers with methacryloylethyl betaine and at least one monomer selected from methacrylic acid and methacrylic acid esters; copolymers with acrylic acid, methyl acrylate and methacrylamidopropyltrimethylammonium chloride; copolymers with acrylamidopropyltrimethylammonium chloride and acrylates; copolymers with acrylamide, acrylamidopropyltrimethylammonium chloride, 2-amidopropylacrylamide sulfonate and dimethylaminopropylamine.

Cationic copolymers can be obtained by copolymerization of the fluorescent monomers with non-fluorescent cationic monomers, whereby also optionally additional non-cationic comonomers can be copolymerized. Cationic monomers are especially those with primary, secondary, tertiary or quaternary amino groups. The cationic charge density will be preferably from 1 to 7 meq/g. Suitable cationic monomers are unsaturated, radically polymerizable compounds and contain preferably quaternary amino groups. The cationic groups can be situated either in the polymer chain or preferably as substituent on one or more monomers. Suitable cationic monomers are especially ammonium-substituted vinyl monomers such as, for example, trialkylammoniumalkyl acrylate, trialkylammoniumalkyl methacrylate, methacrylamidoalkyltrialkylammonium, acrylamidoalkyltrialkylammonium, dialkyldiallylammonium, alkylamine oxide methacrylate, quaternary vinylammonium monomers with cyclic, cationic groups containing nitrogen such as pyridinium, imidazolium or quaternary pyrrolidones, e.g. alkylvinylimidazolium, alkylvinylpyridinium, or alkylvinylpyrrolidone salts. The alkyl groups of these monomers are preferably lower alkyl groups such as, for example, C1 to C7 alkyl groups, and especially preferred are C1 to C3 alkyl groups.

Preferred cationic fluorescent copolymers are copolymers of fluorescent monomers, especially those of the formulae I to III with the following additional non-fluorescent monomers: copolymers with dimethyldiallylammonium chloride; copolymers with acrylamide and dimethyldiallylammonium chloride; copolymers with vinylpyrrolidone and dialkylaminoalkyl methacrylate and quaternization e.g. by reaction with diethyl sulfate to dimethylaminoethyl methacrylate methosulfate; copolymer with methylvinylimidazolium chloride and vinylpyrrolidone; copolymer with trimethylammoniumethyl methacrylate chloride; copolymer with dimethyldiallylammonium chloride, sodium acrylate and acrylamide; copolymer with vinylpyrrolidone, dimethylaminopropyl methacrylamide and methacryloylaminopropyllauryldimethylammonium chloride; copolymer with vinylpyrrolidone, dimethylaminoethylmethacrylate and vinylcaprolactam; copolymer with vinylpyrrolidone and methacrylamidopropyltrimethylammonium chloride; copolymer with vinylpyrrolidone and dimethylaminoethyl methacrylate; copolymers with vinylpyrrolidone, vinylcaprolactam and dimethylaminopropylacrylamide.

A further objekt of the invention are also copolymers containing at least one polymerizable fluorescent monomer, preferably II or III; at least one polymerizable comonomer selected from vinylcaprolactone, vinylcaprolactame, vinylpyrrolidone, aminoalkyl acrylate, aminoalkyl methacrylate, dialkylaminoalkyl acrylate, dialkylaminoalkyl methacrylate, monoalkylamino alkylacrylate, monoalkylaminoalkyl methacrylate, dialkylaminoalkyl acrylamide, dialkylaminoalkyl methacrylamide, alkylaminoxide methacrylate, methacryloylalkylbetaine, methacrylamidoalkyltrialkylammonium, acrylamidoalkyltrialkylammonium, quaternary crotonbetaines, quaternary crotonbetaine esters, trialkylammoniumalkyl methacrylate, trialkylammoniumalkyl acrylate, dialkyldiallylammonium, alkylvinylimidazolium, alkylvinylpyridinium; whereby the alkyl groups of these monomers are preferably C1 to C7 alkyl groups, especially preferred C1 to C3 alkyl groups such as methyl or ethyl; and optinoally further comonomers. The copolymers comprise preferably 0,1 to 10% by weight more preferred 1 to 5% by weight of fluorescent monomers, and about 90 to 99% by weight non-fluorescent comonomers.

The preparation of the homo- and copolymers can be achieved fundamentally with all common polymerization methods for unsaturated ethylenic monomers, whereby the details can be selected in known manner contingent upon the reactivity subject to the substitution pattern of the monomers. The radically initiated polymerization can be carried out in solution, in the solid state, emulsion or suspension. In addition to the usual azo compounds (e.g. azo-bis-isobutyronitrile), organic peroxides (dibenzoyl peroxide) inorganic persulfates (potassium peroxodisulfate) or redox systems (Fe(II)/Fe(III)) can also be used as chain initiators. Further possibilities arise from initiation by high-energy radiation (UV light, radiolysis), electron beam, ultrasound both alone or in combination with the aforementioned radical chain initiators. As a means for the control of monomer distribution and chain length living radical or controlled radical polymerization can be used as a special form of radical polymerization. This is achieved, for example, by reversible termination of the reactive chain terminus by N-oxyls. Ionic polymerization methods can also be used, but are more greatly dependent upon the substitution pattern of the monomers in respect of reactivity. This also applies to polyinsertion methods that are carried out with, for example, Ziegler-Natta catalysts. The methods described in DE 102 33 179 A1 and corresponding analogous methods are preferably used as preparation methods for the fluorescent polymers. At variance to this, noncontinuous fed-batch processes, during which the more reactive, preferably polymerizing monomers are added continuously throughout the polymerization, are used for monomer ratios that cannot be obtained by simple methods owing to reactivity parameters in order to ensure a homogenous polymer composition (e.g. diallyldimethylammonium chloride, DADMAC).

### Hair treatment agents

According to the invention the fluorescent polymers can be used for the treatment of human hair and for the preparation of agents for the treatment of human hair. The hair treatment agents according to the invention can be agents for cleaning hair, for the conditioning of hair or for temporary styling and/or stabilization of the hair style (styling agent) that can be applied in the most varied of methods, e.g. as leave-on or as rinse-off products. Forms of application are, for example, shampoos, deep conditioners, conditioners, hair lotions, repair fluids, hair oils, brilliantines, hair sprays, hair lacquers, setting lotions, hair foams, hair gels, hair waxes, hair creams, etc. Object of the invention is thus also hair treatment agents that are suitable for the treatment of human hair and contain at least one fluorescent polymer in a cosmetically compatible base. Preferred is a hair treatment agent that is present either
- as hair wax containing at least one wax, or
- as deep conditioner or hair lotion in the form of a preparation containing at least one additional hair-conditioning material, or
- as hair gel with at least one gelling agent, or
- as aerosol or pump foam in combination with a device for foaming, or
- as hair spray in combination with a spray device, or
- as hair cream in the form of an o/w- or w/o emulsion, or
- as shampoo containing at least one detersive surfactant.

According to the invention hair cosmetic preparations contain the fluorescent polymers preferably in an amount from 0.01 to 20 percent by weight, especially from 0.1 to 15 percent by weight, or from 0.3 to 10 percent by weight. Dependent upon type and purpose, further active substances and additives are present preferably in an amount from 0.01 to 20 percent by weight, especially from 0.05 to 10 or from 0.1 to 5 percent by weight.

The agent according to the invention can be used to obtain a particular brilliance of the thus treated hair, especially with the use of fluorescent polymers with high quantum yields (e.g. 50-100%). In one embodiment the agent according to the invention is present in the form of a hair wax, i.e. it has a waxy consistency and contains at least one wax in an amount from preferably 0.5 to 30 percent by weight as well as optionally further water-insoluble substances. The waxy consistency is preferably characterized in that the needle penetration number (unit of measurement 0.1 mm (0.004 in), test weight 100 g (3.53 oz), testing time 5 s, test temperature 25° C (77° F); according to DIN 51 579) is greater or equal to 10, especially preferred greater or equal to 20 and that the solidification point of the product is preferably greater or equal to 30° C (86° F) and lower or equal to 70° C (158° F), especially preferred in the range from 40 (104° F) to 55° C (131° F).

In principle, any wax known in the prior art can be used as wax material. These include animal, vegetable, mineral and synthetic waxes, microcrystalline waxes, macrocrystalline waxes, solid paraffins, petrolatum, Vaseline, ozocerite, montanin wax, Fischer-Tropsch wax, polyolefine waxes, e.g. polybutene, beeswax, wool wax and its derivatives such as, for example, wool wax alcohols, candelilla wax, olive wax, carnauba wax, Japan wax, apple wax, hydrogenated fats, fatty acid esters, fatty acid glycerides, wax-like emulsifiers with an HLB value below 7, fatty alcohols, fatty acids or hydrophilic waxes such as, for example, high molecular weight polyethyleneglycols with a molecular weight from 800 to 20,000, preferably from 2,000 to 10,000 g/mol, polyethylene waxes and silicone waxes. The wax materials have a solidification point preferably above 40° C (102° F), especially above 55° C (131° F). The needle penetration number (0.1 (0.004 in), 100 g (3.53 oz), 5 s, 25° C (77° F); according to DIN 1 579) preferably lies in the range from 2 to 70, especially from 3 to 40.

In addition to wax materials, liquid hydrophobic oils may be present. The oils have a melting point of preferably less than or equal to 25° C (77° F) and a boiling point preferably above 250° C (482° F), especially above 300° C (572° F). In principle, any oil generally known to the person skilled in the art can be used for this purpose. Suitable oils are vegetable or animal oils, mineral oils (liquid paraffin), silicone oils or their mixtures. Hydrocarbon oils, e.g. paraffin or isoparaffin oils, squalane, oils from fatty acids and polyols, especially triglycerides are suitable. Suitable vegetable oils are, for example, sunflower oil, coconut oil, castor oil, lanolin oil, jojoba oil, corn oil, soy oil.

In one embodiment the agent according to the invention is present as hair conditioner e.g. as deep conditioner or as hair lotion and contains in addition at least one hair conditioning material. The hair conditioning material is preferably present in an amount from 0.01 to 20, especially preferred 0.05 to 10, most especially preferred from 0.1 to 5 percent by weight. Hair conditioning materials are selected, for example, from cationic surfactants; cationic polymers; betaine; panthenol; panthenyl ethyl ether; sorbitol; protein hydrolysates; plant extracts or one of the aforementioned oils or waxes.

Suitable cationic surfactants contain amino groups or quaternized hydrophilic ammonium groups that carry a positive charge in solution and can be represented by the general formula

N⁽⁺⁾R¹R²R³R⁴ X⁽⁻⁾

whereby R1 to R4 independently from one another stand for aliphatic groups, aromatic groups, alkoxy groups, polyoxyalkylene groups, alkylamido groups, hydroxyalkyl groups, aryl groups or alkaryl groups with 1 to22 C atoms, whereby at least one residue has at least 6, preferably at least 8 C atoms and X- represents an anion, for example, a halide, acetate, phosphate, nitrate or alkyl sulfate, preferably a chloride. In addition to the C atoms and hydrogen atoms, the aliphatic groups can also contain crosslinks or other groups such as, for example, further amino groups. Examples of suitable cationic surfactants are the chlorides or bromides of alkyldimethylbenzylammonium salts, alkyltrimethylammonium salts, e.g. cetyltrimethylammonium chloride or bromide, tetradecyltrimethylammonium chloride or bromide, alkyldimethylhydroxyethylammonium chlorides or bromides, dialkyldimethylammonium chlorides or bromides, alkylpyridinium salts, for example, lauryl- or cetylpyridinium chloride, alkylamidoethyltrimethylammonium ether sulfates as well as compounds with cationic character such as amine oxides, e.g. alkylmethylamine oxides or alkylaminoethyldimethylamine oxides. Especially preferred are C8-22 alkyldimethylbenzylammonium compounds, C8-22 alkyltrimethylammonium compounds, especially cetyltrimethylammonium chloride, C8-22 alkyldimethylhydroxyethylammonium compounds, di-(C8-22 alkyl)-dimethylammonium compounds, C8-22 alkylpyridinium salts, C8-22 alkylamidoethyltrimethylammonium ether sulfates, C8-22 alkylmethylamine oxides, C8-22 alkylaminoethyldimethylamine oxides.

In addition to the aforementioned cationic surfactants, other suitable cationic or amino-substituted surfactants are those of the formula R1-NH-(CH₂)n-NR2R3 or of the formula R1-NH-(CH₂)n-N⁺R2R3R4 X, wherein R1 is an acyl or an alkyl residue with 8 to 24 C atoms, which can be branched or linear, saturated or unsaturated, whereby the acyl and/or the alkyl residue can contain one or more OH groups, R2, R3 and R4 independently of one another are hydrogen, alkyl or alkoxyalkyl residues with 1 to 6 C atoms, which can be same or different, saturated or unsaturated and can be substituted with one or more hydroxy groups, X⁻ is an anion, especially a halide ion or a compound of the general formula RSO₃⁻, wherein R has the meaning of saturated or unsaturated alkyl residues with 1 to 4 C atoms, und n means a whole number between 1 und 10, preferably from 2 to 5.

The active hair conditioning compound is preferably an amidoamine and/or a quaternized amidoamine of the aforementioned formulae, wherein R1 is a branched or linear, saturated or unsaturated acyl residue with 8 to 24 C atoms that can contain at least one OH group. Preferred are such amines and/or quaternized amines, in which at least one of the residues R2, R3 and R4 means a residue according to the general formula CH₂CH₂OR5, wherein R5 can have the meaning of alkyl residues with 1 to4 C atoms, hydroxyethyl or H. Suitable amines or amidoamines, which can be optionally quaternized, are especially such with the INCI names ricinoleamidopropyl betaines, ricinoleamidopropyl dimethylamine, ricinoleamidopropyl dimethyl lactate, ricinoleamidopropyl ethyldimonium ethosulfate, ricinoleamidopropyltrimonium chloride, ricinoleamidopropyltrimonium methosulfate, cocamidopropyl betaine, cocamidopropyl dimethylamine,cocamidopropyl ethyldimonium ethosulfate, cocamidopropyltrimonium chloride, behenamidopropyl dimethylamine, isostearylamidopropyl dimethylamine, stearylamidopropyl dimethylamine, quatemium-33, undecyleneamidopropyltrimonium methosulfate.

In one embodiment the agent according to the invention contains as hair conditioning or hair setting additive at least one cationic polymer, i.e. a polymer with cationic or cationizable groups, especially primary, secondary, tertiary or quaternary amine groups in an amount preferably from 0.01 to 20 percent by weight or from 0.05 to 10 percent by weight, especially preferred from 0.1 to 5 percent by weight. The cationic charge density is preferably 1 to 7 meq/g.

The suitable cationically active polymers are preferably hair setting or hair conditioning polymers. Suitable polymers contain preferably quaternary amine groups. Cationic polymers can be homo- or copolymers, where the quaternary nitrogen groups are contained either in the polymer chain or preferably as substituents on one or more of the monomers. The monomers containing ammonium groups can be copolymerized with non-cationic monomers. Suitable cationic monomer are unsaturated compounds that can undergo radical polymerization, which bear at least one cationic group, especially ammonium-substituted vinyl monomers such as, for example, trialkylmethacryloxyalkylammonium, trialkylacryloxyalkylammonium, dialkyldiallylammonium and quaternary vinylammonium monomers with cyclic, cationic nitrogen-containing groups such as pyridinium, imidazolium or quaternary pyrrolidones, e.g. alkylvinylimidazolium, alkylvinylpyridinium, or alkylvinylpyrrolidone salts. The alkyl groups of these monomers are preferably lower alkyl groups such as, for example, C1 to C7 alkyl groups, especially preferred are C1 to C3 alkyl groups.

The monomers containing ammonium groups can be copolymerized with non-cationic monomers. Suitable comonomers are, for example, acrylamide, methacrylamide, alkyl- and dialkylacrylamide, alkyl- and dialkylmethacrylamide, alkyl acrylate, alkyl methacrylate, vinylcaprolactone, vinylcaprolactam, vinylpyrrolidone, vinyl esters, for example, vinyl acetate, vinyl alcohol, propylene glycol or ethylene glycol, whereby the alkyl groups of these monomers are preferably C1 to C7 alkyl groups, and especially preferred are C1 to C3 alkyl groups.

Suitable polymers with quaternay amine groups are, for example, the polymers described in the CTFA Cosmetic Ingredient Dictionary under the names polyquaternium such as methylvinylimidazolium chloride/vinylpyrrolidone copolymer (Polyquaternium-16) or quaternized vinylpyrrolidone/dimethylaminoethyl methacrylate copolymer (Polyquaternium-11) as well as quaternary silicon polymers or oligomers such as, for example, silicon polymers with quaternary end groups (Quaternium-80).

Preferred cationic polymers of synthetic origin: poly(dimethyldiallylammonium chloride); copolymers from acrylamide and dimethyldiallylammonium chloride; quaternary ammonium polymers formed by the reaction of diethyl sulfate and a copolymer from vinylpyrrolidone and dimethylaminoethyl methacrylate, especially vinylpyrrolidone/ dimethylaminoethyl methacrylate methosulfate copolymer (e.g. Gafquat^{®} 755 N, Gafquat^{®} 734); quaternary ammonium polymers from methylvinylimidazolium chloride and vinylpyrrolidone (e.g. LUVIQUAT^{®} HM 550); Polyquaternium-35; Polyquaternium-57; polymer from trimethylammoniumethyl methacrylate chloride; terpolymers from dimethyldiallylammonium chloride, sodium acrylate and acrylamide (e.b. Merquat^{®} Plus 3300); copolymers from vinylpyrrolidone, dimethylaminopropylmethacrylamide and methacryloylaminopropyllauryldimethylammonium chloride; terpolymers from vinylpyrrolidone, dimethylaminoethyl methacrylate and vinylcaprolactam (e.g. Gaffix^{®} VC 713); vinylpyrrolidone/methacrylamidopropyltrimethylammonium chloride copolymers (e.g. Gafquat^{®} HS 100); copolymers from vinylpyrrolidone and dimethylaminoethyl methacrylate; copolymers from vinylpyrrolidone, vinylcaprolactam and dimethylaminopropylacrylamide; poly- or oligoesters, constructed from at least a first type of monomer that is selected from a hydroxy acid substituted with at least one quaternary ammonium group; dimethylpolysiloxanes substituted terminally with quaternary ammonium groups.

Suitable cationic polymers that are derived from natural polymers are especially cationic derivatives of polysaccharides, for example, cationic derivatives of cellulose, starch or guar. Furthermore, chitosan and chitosan derivatives are also suitable. Cationic polysaccharides are, for example, represented by the general formula

G-O-B-N⁺R^{a}R^{b}R^{c} X⁻

G is an anhydroglucose residue, for example, starch or cellulose anhydroglucoses;
B is a divalent linking group, for example, alkylene, oxyalkylene, polyoxyalkylene or hydroxyalkylene;
R^{a}, R^{b} and R^{c} are independently from one another alkyl, aryl, alkylaryl, arylalkyl, alkoxyalkyl or alkoxyaryl, any of which can have up to 18 C atoms, whereby the total number of C atoms in R^{a}, R^{b} and R^{c} is preferably a maximum of 20;
X is a conventional counter-anion, for example, a halide, acetate, phosphate, nitrate, or alkyl sulfate, preferably a chloride. Cationic celluloses are, for example, those with the INCI names Polyquaternium-10 or Polyquaternium-24. A suitable cationic guar derivative has, for example, the INCI name guar hydroxypropyltrimonium chloride.

Especially preferred cationically-active substances are chitosan, chitosan salts and chitosan derivatives. Chitosans that can be used according to the invention can be fully or partially deacetylated chitins. The molecular weight can be distributed over a wide range, for example, from 20,000 to ca. 5 million g/mol, e.g. from 30,000 to 70,000 g/mol. However, the molecular weight will preferably lie above 100,000 g/mol, and especially preferred from 200,000 to 700,000 g/mol. The degree of deacetylation is preferably 10 to 99%, especially preferred 60 to 99%. A preferred chitosan salt is chitosonium pyrrolidone carboxylate, e.g. Kytamer® PC with a molecular weight of ca. 200,000 to 300,000 g/mol and deacetylation of 70 to 85%. Suitable chitosan derivates are quaternized, alkylated or hydroxyalkylated derivates. e.g. hydroxyethyl-, hydroxypropyl- or hydroxybutylchitosan. The chitosans or chitosan derivatives are preferably present in their neutralized or partially neutralized form. The degree of neutralization will be preferably at least 50%, especially preferably between 70 and 100%, as calculated on the basis of the number of free base groups. For the neutralization agent, in principle any cosmetically compatible inorganic or organic acids can be used such as, for example, formic acid, tartaric acid, malic acid, lactic acid, citric acid, pyrrolidone carboxylic acid, hydrochloric acid and others, of which pyrrolidone carboxylic acid is especially preferred.

Preferred cationic polymers derived from natural sources:
cationic cellulose derivates from hydroxyethylcellulose and diallyldimethylammonium chloride; cationic cellulose derivates from hydroxyethylcellulose and trimethylammonium-substituted epoxide; chitosan and its salts; hydroxyalkyl chitosans and their salts; alkylhydroxyalkyl chitosans and their salts; N-hydroxyalkylchitosan alkyl ethers.

In one embodiment the agent according to the invention is in the form a gel, a viscous lotion or in the form of a spray gel that is sprayed with a mechnical device and contains at least one gelling agent, preferably at least one gel-forming thickening polymer. The gelling agent is present in an amount of preferably 0.05 to 10, especially preferred from 0.1 to 5 percent by weight. The gel-like or viscous product has a viscosity of preferably at least 250 mPa s (measured with a Bohlin Rheometer CS, gaging member C25 at 25° C (77° F) and a shear rate of 50 s⁻¹). The viscosity of the gel is preferably from 500 to 50,000 mPa s, especially from 1,000 to 15,000 mPa s at 25° C (77° F).

Gelling agents are selected, for example, from copolymers from at least one first type of monomer selected from acrylic acid and methacrylic acid and at least one second type of monomer selected from esters of acryl acid and ethoxylated fatty alcohol; cross-linked polyacrylic acid; cross-linked copolymers from at least one first type of monomer selected from acrylic acid and methyacrylic acid and at least one second type of monomer selected from esters of acrylic acid with C10 to C30 alcohols; copolymers from at least one first type of monomer selected from acrylic acid and methacrylic acid and at least one second type of monomer selected from esters of itaconic acid and ethoxylated fatty alcohol; copolymers from at least one first type of monomer selected from acrylic acid and methacrylic acid, at least one second type of monomer selected from esters of itaconic acid and ethoxylated C10 to C30 alcohols, and a third type of monomer selected from C1 to C4 aminoalkylacrylates; copolymers from two or more monomers selected from acrylic acid, methacrylic acid, esters of acrylic acid and methacrylic acid; copolymers from vinylpyrrolidone and ammoniumacryloyldimethyl taurate; copolymers from ammoniumacryloyldimethyl taurate and monomers selected from esters of methacrylic acid and ethoxylated fatty alcohols; hydroxyethylcellulose; hydroxypropylcellulose; hydroxypropylguar; glyceryl polyacrylate; glyceryl polymethacrylate; copolymers from at least one C2, C3 or C4 alkylene and styrene; polyurethanes; hydroxypropylstarch phosphate; polyacrylamide; copolymers from maleic anhydride and methyl vinyl ether cross-linked with decadiene; carob bean flour; guar; xanthan; dehydroxanthan; carrageenan; karaya gum; hydrolyzed corn starch; copolymers from polyethylene oxide, fatty alcohols and saturated methylenediphenyldiisocyanate (e.g. PEG-150/stearyl alcohol/ SMDI copolymer).

In one embodiment the agent according to the invention is present in the form of a foamable product (mousse) in combination with a device for foaming. It contains at least one known conventional foaming agent, e.g. at least one foam-forming surfactant or at least one foam-forming polymer. Devices for foaming are understood to be devices that enable the foaming of a liquid either with or without the use of an aerosol propellant. As a suitable mechanical foaming device, for example, a commercially available foam pump or an aerosol foam head can be used. The product is present either in combination with a mechanical foam pump device (pump foam) or in combination with at least one aerosol propellant (aerosol pump) in an amount from preferably 1 to 20, especially from 2 to 10 percent by weight. Aerosol propellants are selected from, for example, propane, butane, dimethyl ether and fluorinated hydrocarbons. The agent is foamed immediately before use and is worked into the hair as a foam and can then be rinsed out or can be left in the hair without rinsing.

In one embodiment the agent according to the invention is present in the form of a spray product (hair spray) in combination with a spray device. It can be a pump spray when the spraying device is a mechanical pump spraying device. It can also be an aerosol spray when the agent is present in combination with a pressure-proof container, a spray head, and at least one aerosol propellant selected from propane, butane, dimethyl ether and fluorinated hydrocarbons. An aerosol spray contains in addition preferably 15 to 85 percent by weight, especially preferred 25 to 75 percent by weight of an aerosol propellant and is charged into a pressurized container. Suitable aerosol propellants are, for example, lower alkanes such as, for example, n-butane, i-butane and propane, or also their mixtures as well as dimethyl ether or fluorohydrocarbons such as F 152a (1,1-difluoroethane) or F 134 (tetrafluoroethane) as well as other pressurized gaseous aerosol propellants such as, for example, N₂, N₂O and CO₂ as well as mixtures of the aforementioned aerosol propellants.

A non-aerosol hairspray is sprayed with the help of a suitable mechanically operated spraying device. A mechanical spraying device is understood to be a device that enables the spraying of a composition without the use of a aerosol propellant. A suitable mechanical spraying device can be, for example, a spray pump or a flexible container fitted with a spray valve, in which the agent according to the invention is charged under pressure when the flexible container expands, and from which the agent is released continuously as a result of contraction of the flexible container on opening the spray valve.

The agent of the invention can also exist as a shampoo in combination with at least one detersive surfactant, preferably at least one anionic, amphoteric or non-ionic surfactant. The detersive surfactant is present in an amount from preferably 1 to 50 percent by weight, preferably from 3 to 30 percent by weight, especially from 5 to 20 percent by weight.

Suitable anionic surfactants are, for example, salts and esters of carboxylic acids, alkyl ether sulfates and alkyl sulfates, fatty alcohol ether sulfates, sulfonic acids and their salts (e.g. sulfosuccinates or fatty acid isethienates), phosphoric acid esters and their salts, acylamino acids and their salts. A comprehensive description of these anionic surfactants is found in the publication "FIEDLER - Lexikon der Hilfsstoffe", volume 1, fifth edition (2002), pages 97 to 102, to which expressed reference is made. Suitable anionic surfactants are selected, for example, from the alkali or alkaline earth salts of C10 to C18 alkyl sulfates, the C10 to C18 alkyl sulfonates, the C10 to C18 alkylbenzene sulfonates, the C10 to C18 xylene sulfonates and the C10 to C18 alkyl ether sulfates ethoxylated with 1 to 10 ethylene oxide units, the ethoxylated sulfosuccinic acid half esters of the formula

R¹(OCH₂CH₂)ₘ-O₂C-CH₂CH(SO₃M)-CO₂M,

whereby R1 means a C10 to C18 alkyl residue, M represents an alkali or alkaline earth cation, and m means a whole number from 1 to 10; the alkyl ether carboxylates of the formula R²(OCH₂CH₂)ₙ-OCH₂COOM, whereby R2 means a C10 to C18 alkyl residue, M represents an alkali or alkaline earth cation and n means a whole number of 1 to 20. Preferred surfactants are alkyl ether sulfates, alkyl sulfates and alkyl sulfonates, whereby the alkali and alkaline earth salts of C10 to C18 alkyl ether sulfates ethoxylated with 1 to 10 ethylene oxide units are especially preferred, especially sodium lauryl ether sulfate. Of the alkyl sulfates sodium lauryl sulfate is preferred. Of the suitable alkyl sulfonates the sodium salts of the secondary C12 to C16 alkane sulfonates and their mixtures are preferred. Of the suitable alkylbenzene sulfonates the sodium salt of the linear dodecylbenzene sulfonate is preferred. Of the suitable alkyl ether sulfates those are preferred that possess a C12 to C 16 alkyl, preferably a lauryl, residue and is ethoxylated with 2 to 4, preferably 3 ethylene oxide units (INCI: sodium laurethsulfate). Of the suitable ethoxylated sulfosuccinic acid half esters preferred is that which is ethoxylated with 2 to 4, preferably 3, ethylene oxide units and posseses a C12 to C16 alkyl, preferably a lauryl, residue. Of the alkyl ether carboxylates preferred is that which is ethoxylated with 8 to 14, preferably 10, ethylene oxide units and has a C12 to C16 alkyl, preferably a lauryl, residue.

Suitable non-ionic surfactants are, for example,
- ethoxylated fatty alcohols, fatty acids, fatty acid glycerides or alkyl phenols, especially addition products of 2 to 30 mol ethylene oxide and/or 1 to 5 mol propylene oxide to C8 to C22 or C12 to C18 fatty alcohols, to C12 to C22 fatty acids or to alkylphenols with 8 to 15 C atoms in the alkyl group
- C12 to C22 fatty acid mono- and diesters of addition products of 1 to 30 mol ethylene oxide to glycerol
- addition products of 5 to 60 mol ethylene oxide to castor oil or hydrogenated castor oil
- fatty acid sugar esters, especially esters from saccharose and one or two C8 to C22 fatty acids, INCI: sucrose cocoate, sucrose dilaurate, sucrose distearate, sucrose laurate, sucrose myristate, sucrose oleate, sucrose palmitate, sucrose ricinoleate, sucrose stearate
- esters from sorbitan and one, two or three C8 to C22 fatty acids and a degree of ethoxylation of 4 to 20
- polyglyceryl fatty acid esters, especially from one, two or more C8 to C22 fatty acids and polyglycerol with preferably 2 to 20 glyceryl units
- fatty acid alkanolamides
- alkylglucosides, alkyloligoglucosides and alkylpolyglucoside with C8 to C22 alkyl groups, e.g. decyl glucoside or lauryl glucoside.

Suitable amphoteric surfactants are, for example, derivates of aliphatic quaternary ammonium, phosphonium and sulfonium compounds of the formula 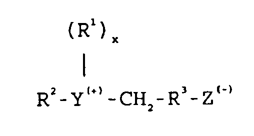 whereby R2 represents a linear or branched alkyl, alkenyl or hydroxyalkyl group with 8 to 18 C atoms and 0 to about 10 ethylene oxide units and 0 to 1 glyceryl units; Y is an N, P or S atom; R1 is an alkyl or monohydroxyalkyl group with 1 to 3 C atoms; X equals 1 if Y is a sulfur atom and X equals 2 if Y is a nitrogen atom or a phosphorus atom; R3 is an alkylene or hydroxyalkylene group with 1 to 4 C atoms and Z⁽⁻⁾ represents a carboxylate, sulfate, phosphonate or phosphate group.

Other amphoteric surfactants, especially betaines are also suitable for the hair detersive agent according to the invention. Examples of betaines include C8 to C18 alkylbetaines such as cocodimethylcarboxymethylbetaine, lauryldimethylcarboxymethylbetaine, lauryldimethyl-alphacarboxyethylbetaine, cetyldimethylcarboxymethylbetaine, oleyldimethylgammacarboxypropylbetaine and laurylbis-(2-hydroxypropyl)-alpha-carboxyethylbetaine; C8 to C18 sulfobetaines such as cocodimethylsulfopropylbetaine, stearyldimethylsulfopropylbetaine, lauryldimethylsulfoethylbetaine, lauryl-bis-(2-hydroxyethyl)sulfopropylbetaine; the carboxyl derivates of imidazole, C8 to C18 alkyldimethylammonium acetate, C8 to C18 alkyldimethylcarbonylmethylammonium salts as well as C8 to C18 fatty acid alkylamidobetaines such as, for example, coconut acid amidopropylbetaine (INCI: cocamidopropylbetaine) and N-coconut oil fatty acid amidoethyl-N-[2-(carboxymethoxy)ethyl]-glycerol (INCI: cocoamphocarboxyglycinate). Especially preferred is a surfactant mixture of lauryl ether sulfate and cocamidopropylbetaine.

The following examples should serve to illustrate further the object of the present invention.

### Examples

### 1.) Monomers

### 1a) Synthesis of 1-(4-vinylphenyl)-1,6-dihydroisoindolo[6,7,1-cde]indole-2,5-dione (c)

A mixture of 2.92 g (0.103 oz) (0.01 mol) 6-bromo-2-oxo-1,2-dihydrobenzo[*cd*]indole-5-carboxylic acid (a), 1.19 g (0.042 oz) (0.01 mol) 4-vinylaniline (b), 1.38 g (0.049 oz) anhydrous potassium carbonate (0.01 mol) and 1.81 g (0.064 oz) (0.01 mol) copper acetate are stirred in 10 ml (0.11 in³) DMF under reflux for three hours. After cooling the batch is transferred to 200 ml (12.2 in³) 3N hydrochloric acid, stirred for a further 15 minutes at room temperature and allowed to stand overnight. The precipitate is filtered off, washed with a little water and dried in vacuum. Purification is carried out by column chromatography on neutral alumina. The crude product is dissolved in a little DMF, applied to the column and eluted with ethanol. The product fraction is concentrated, the dye (c) is precipitated with water and recrystallized from ethanol. m.p. (c): >280° C (536° F), pink-colored crystals

### 1b) Synthesis of N¹-(1-propene-3-yl)-[N²,N³-bis(1-hexylheptyl)-benzo[ghi]perylene-2,3,8,9,11,12-hexacarbocylic acid-1,3:8,9:11,12-tris-(dicarboximide) (f)

0.25 g (0.009 oz) (0.29 ol) N²,N³-Bis(1-hexylheptyl)-benzo[ghi]perylene-2,3,8,9,11,12-hexacarboxylic acid-2,3:8,9:11,12-tris(dicarboximide) (d), 0.11 g (0.004 oz) (0.87 mmol) allyl bromide (e) and 1.04 g (0.037 oz) (7.54 mmol) anhydrous potassium carbonate are homogenized and suspended in 40 ml (2.4 in³) absolute DMF. The reaction mixture is now stirred at 100° C (212°F) for 24 hours. After cooling the batch is diluted with 100 ml (6.1 in³) water, the precipitated crude product is filtered off, washed with water and dried in vacuum. After purification by chromatography on silica with chloroform as eluent the dye N¹-(1-propen-3-yl)-[N²,N³-bis(1-hexylheptyl)-benzo[ghi]perylene-2,3,8,9,11,12-hexacarboxylic acid-2,3:8,9:11,12-tris-(dicarboximide) (f) is obtained as a luminous yellow powder. m.p. (f): >300° C(572° F), luminous yellow powder

### 2.) Polymers

- S =: styrene
- LMA =: lauryl methacrylate
- TBMA =: tert-butyl methacrylate
- VP =: vinylpyrrolidone
- VA =: vinyl acetate
- DMAPMA =: 3-(N,N-dimethyl)aminopropyl methacrylate
- DADMAC =: diallyldimethylammonium chloride
- VCL =: Vinyl caprolactone
- M1a: fluorescent monomer from example 1a
- M1b: fluorescent monomer from example 1b

| No. | M1a | Mlb | S | LMA | TBMA | VP | VA | DMAPMA | DADMAC | VCL | Color |
|---|---|---|---|---|---|---|---|---|---|---|---|
| 1 | 45 | | 15 | | | 40 | | | | | pink |
| 2 | 35 | | | 25 | | | | 40 | | | pink |
| 3 | 35 | | | 25 | | | | 33 | | 7 | pink |
| 4 | 31 | | 8 | | 27 | | | | 14 | 20 | pink |
| 5 | 25 | 12 | | 25 | | | | 38 | | | red |
| 6 | 38 | | 12 | | | 26 | | 5 | | 19 | pink |
| 7 | 12 | 30 | | | 34 | | 24 | | | | orange |
| 8 | 39 | 15 | | | | | | 23 | | 23 | red |
| 9 | 12 | 40 | | | | | | 25 | | 23 | orange |
| 10 | 12 | 35 | 12 | | | 19 | | 8 | | 14 | orange |
| 11 | | 51 | 15 | | | | | | | 34 | yellow |
| 12 | 5 | 43 | | 25 | | | | 20 | | 7 | golden yellow |
| 13 | 10 | 25 | 12 | | | 29 | | 5 | | 19 | orange |
| 14 | | 32 | 24 | | 5 | | | | | 39 | yellow |
| 15 | 27 | 23 | | 10 | | 15 | 25 | | | | red |

Advantageous properties from the use in cosmetic hair care products
- Non-permanent color effect on light hair
- Advantage over pigments:
   no separation even in solutions without yield point (e.g. aerosol sprays)
- even application to human hair
- no change in viscosity through solids content
- no problems with sprayability (special valves not required even with aerosols)
- dyes are of high molecular weight, no penetration into the skin/body
- application can be carried out in the dissolved or suspended state.

### Polymer Examples 16 A and B

Monomer composition for producing polymer 16A:
5% N-(1-Hexyl-heptyl)-N'-(4-vinylphenyl)-perylen-3,4:9,10-bis(dicarboximid)
60% acrylic acid
35% dimethylaminoethyl methacrylate

Monomer composition for producing polymer 16B:
5% N-(1-Hexyl-heptyl)-N'-(4-vinylphenyl)-perylen-3,4:9,10-bis(dicarboximid)
60% acrylic acid
35% vinyl pyrrolidone

Monomers are dissolved in dioxane with a total concentration of monomers of 0,5 mol/l under a nitrogen atmosphere. At a reaction temperature of 60°C the starter (AIBN) is added as 0,01 mol/l solution in dioxane. The reaction can be monitored by taking samples at time intervals selected according to the reaction rate. The samples can be weighed and the resulting polymer precipitated in methanol.

The polymerisation can also be made under nitrogen in toluene at 60°C using AIBN as starter. After about 2 hours the resulting polymer precipitates and can be isolated by filtering off.

Both polymers 16 A and B are intensively red colored polymers which show reddish-orange fluorescence at an emission wave length of 534 nm. They can be dissolved in aequeous hair care formulations by neutralizing the acid groups with a base. Polymer B is soluble in ethanol an can be used e.g. in alcoholic hair fixing lotions of alcoholic or aqueous-alcoholic hair sprays. Polymer A is water-soluble and can be used e.g. in aqueous hair styling gels. It adheres very good on hair and simultaneously has a hair-fixing and hair-coloring effect. The polymers can be washed out of the hair withou leaving residues. An advantage over other hair coloring products is the ease of achieving a homogeneous distribution within the formulation and effectivly preventing color separation.

### Hair cosmetic examples

### Example 1: liquid gel

| | |
|---|---|
| Polymer no. 3 or 16A or 16B | 1.0 g (0.04 oz) |
| PEG-12 dimethicone | 1.5 g (0.05 oz) |
| Carbomer | 0.3 g (0.01 oz) |
| AMP-95 | 0.3 g (0.01 oz) |
| PPG-1-PEG-9 lauryl glycol ether | 0.2 g (0.007 oz) |
| Perfume | 0.15 g (0.005 oz) |
| Hydroxyethylcellulose | 0.4 g (0.01 oz) |
| Ethanol | 16.5 g (0.582 oz) |
| Water | balance to 100g (3.53 oz) |

### Example 2: liquid gel

| | |
|---|---|
| Polymer no. 2 or 16A or 16B | 3.0 g (0.11 oz) |
| Vinylpyrrolidone/vinyl acetate copolymer | 1.0 g (0.04 oz) |
| PEG-12 dimethicone | 1.0 g (0.04 oz) |
| Xanthan gum | 1.2 g (0.04 oz0 |
| Citric acid | 0.1 g (0.004 oz) |
| Perfume | 0.15 g (0.005 oz) |
| Ethanol | 6.5 g |
| DMDM hydantoin | 0.3g (0.01 oz) |
| Water | balance to 100g (3.53 oz) |

### Example 3: liquid gel

| | |
|---|---|
| Polymer no. 12 or 16A or 16B | 2.5 g (0.09 oz) |
| Glucose | 5.0 g (0.18 oz) |
| Propylene glycol | 3.8 g (0.13 oz) |
| Hydroxy propyl guar | 0.3 g (0.01 oz) |
| AMP-95 | 0.2 g (0.007 oz) |
| PEG-25 PABA | 0.5 g (0.02 oz) |
| PEG-40 hydrogenated castor oil | 0.18 g (0.006 oz) |
| PPG-1-PEG-9 lauryl glycol ether | 0.18 g (0.006 oz) |
| Perfume | 0.15 g (0.005 oz) |
| Ethanol | 16.5 g (0.582 oz) |
| Water | balance to 100g (3.53 oz) |

### Example 4: spray gel

| | |
|---|---|
| Polymer no. 15 or 16A or 16B | 2.5 g (0.09 oz) |
| VP/VA COPOLYMER (Luviskol® VA 64) | 3.0 g (0.11 oz) |
| Ethanol | 18 g (0.63 oz) |
| Aminomethylpropanol 95% | 0.1 g (0.004 oz) |
| PEG-40 hydrogenated castor oil | 0.2 g (0.007 oz) |
| Perfume | 0.2 g (0.007 oz) |
| Aculyn® 48 ¹⁾ | 0.5 g (0.02 oz) |
| Water | balance to 70 g (2.5 oz) |

| | |
|---|---|
| ¹⁾ PEG-150/stearyl alcohol/SMDI copolymer, 19% in water | |

The composition is charged into a container with spray pump device.

### Example 5: rapid drying gel

| | |
|---|---|
| Polymer no. 13 or 16A or 16B | 3.2 g (0.11 oz) |
| Aquaflex® SF 40 ¹⁾ | 0.8 g (0.03 oz) |
| PEG-12 dimethicone | 1.5 g (0.05 oz) |
| Acrylates/C10-30 alkyl acrylate crosspolymer | 0.35 g (0.012 oz) |
| AMP-95 | 0.26 g (0.009 oz) |
| Methyl methoxycinnamate | 0.30 g (0.011 oz) |
| Perfume | 0.30 g (0.011 oz) |
| Ethanol | 34.2 g (1.21 oz) |
| Water | balance to 100 g (3.53 oz) |

| | |
|---|---|
| ¹⁾ VP/vinylcaprolactam/DMAPA acrylates copolymer, 40% in ethanol | |

### Example 6: spray gel

| | |
|---|---|
| Polymer no. 15 or 16A or 16B | 2.0 g (0.007 oz) |
| PEG-12 dimethicone | 1.0 g (0.04 oz) |
| Carbomer (Carbopol®) | 0.23 g (0.008 oz) |
| AMP-95 | 0.22 (0.007 oz) |
| Perfume | 0.15 g (0.005 oz) |
| Ethanol | 6.5 g (0.23 oz) |
| Water | balance to 100 g (3.53 oz) |

The composition is charged into a container with spray pump device.

### Example 7: blow dry gel

| | |
|---|---|
| Polymer no. 8 or 16A or 16B | 1.0 g (0.04 oz) |
| PEG-12 dimethicone | 1.0 g (0.04 oz) |
| Hydroxypropylcellulose (Klucel® HF) | 0.95 g (0.034 oz) |
| Citric acid | 0.1 g (0.004 oz) |
| Perfume | 0.15 g (0.005 oz) |
| Ethanol | 6.5 g (0.23 oz) |
| Water | balance to 100 g (3.53 oz) |

### Example 8: gel

| | |
|---|---|
| Polymer no. 5 or 16A or 16B | 2.4 g (0.08 oz) |
| Polyvinylpyrrolidone K 90 | 1.8 g (0.06 oz) |
| PEG-12 dimethicone | 1.5 g (0.05 oz) |
| Synthalen® W 2000 ¹⁾ | 1.0 g (0.04 oz) |
| AMP-95 | 0.3 g (0.01 oz) |
| PEG-25 PABA (Uvinul® P 25) | 0.3 g (0.01 oz) |
| Panthenol | 0.15 g (0.005 oz) |
| Perfume | 0.3 g (0.01 oz) |
| Ethanol | 34,. g (1.14 oz) |
| Keratin hydrolysate | 0.1 g (0.004 oz) |
| Water | balance to 100 g (3.53 oz) |

| | |
|---|---|
| ¹⁾ Acrylates/palmeth-25 acrylate copolymer | |

### Example 9: gel - firm hold

| | |
|---|---|
| Polymer no. 7 or 16A or 16B | 4.5 g (0.16 oz) |
| VA/CROTONATES COPOLYMER (Luviset® CA 66) | 2.5 g (0.09 oz) |
| Sorbitol | 4.2 g (0.15 oz) |
| Carbomer (Tego carbomer 140) | 0.8 g (0.03 oz) |
| AMP-95 | 0.3 g (0.01 oz) |
| Methylparaben | 0.2 g (0.007 oz) |
| PEG-40 hydrogenated castor oil | 0.2 g (0.007 oz) |
| Panthenol | 0.1 g (0.004 oz) |
| Perfume | 0.2 g (0.007 oz) |
| Ethanol | 5.0 g (0.18 oz) |
| Water | balance to 100 g (3.53 oz) |

### Example 10: gel firm hold

| | |
|---|---|
| Polymer no. 7 or 16A or 16B | 5.0 g (0.18 oz) |
| Aquaflex® SF 40 ¹⁾ | 1.5 g (0.05 oz) |
| Vinyl acetate/crotonic acid copolymer | 1.2 g (0.04 oz) |
| Sorbitol | 4.2 g (0.15 oz) |
| Structure® 3001²⁾ | 0,12 g (0.004 oz) |
| AMP-95 | 0.35 g (0.012 oz) |
| PEG-25 PABA | 0.5 g (0.02 oz) |
| Dekaben® LMB | 0.2 g (0.007 oz) |
| PEG-40 hydrogenated castor oil | 0.2g (0.007 oz) |
| Panthenol | 0.1 g (0.004 oz) |
| Perfume | 0.2 g (0.007 oz) |
| Ethanol | 5.0 g (0.18 oz) |
| Water | balance to 100 g (3.53 oz) |

| | |
|---|---|
| ¹⁾ VP/vinylcaprolactam/DMAPA acrylates copolymer, 40% in ethanol ²) Acrylates/Ceteth-20 itaconate copolymer, 30% in water | |

### Example 11: gel - normal hold

| | |
|---|---|
| Polymer no. 3 or 16A or 16B | 3.5 g (0.12 oz) |
| Glycerol | 5.2 g (0.18 oz) |
| Propylene glycol | 4.0 g (0.14 oz) |
| Ammmonium acryloyldimethyl taurate/VPcopolymer (Aristoflex® AVC) | 0.35 g (0.012 oz) |
| AMP-95 | 0.26 g (0.009 oz) |
| Polysorbate-40 | 1.0 g (0.04 oz) |
| Methylparaben | 0.2 g (0.007 oz) |
| PEG-25 PABA | 0.5 g (0.02 oz) |
| Perfume | 0.2 g (0.007 oz) |
| Ethanol | 4.5 g (0.16 oz) |
| Water | balance to 100 g (3.53 oz) |

### Example 12: pump setting foam

| | |
|---|---|
| Poylmer no. 4 or 16A or 16B | 1.9 g (0.07 oz) |
| Vinyl acetate/crotonic acid copolymer | 0.3 g (0.01 oz) |
| Cocamidopropyl hydroxysultaine | 0.4 g (0.01 oz) |
| Citric acid | 0.1 g (0.004 oz) |
| Ethanol | 8.9 g (0.31 oz) |
| Betaine | 0.1g (0.004 oz) |
| Perfume | 0.15 g (0.005 oz) |
| Water | balance to 100 g (3.53 oz) |

The composition is charged into a container with a mechanically driven foam pump device.

### Examples 13: pump setting foam

| | |
|---|---|
| Polymer no. 5 or 16A or 16B | 2.1 g (0.07 oz) |
| Acryl acid/ethyl acrylate/N-tert-butylacrylamide copolymer | 0.4 g (0.01 oz) |
| Cocamidopropyl hydroxysultaine | 0.4 g (0.01 oz) |
| Citric acid | 0.1 g (0.004 oz) |
| Dekaben® LMP | 0.2 g (0.007 oz) |
| Camomile extract | 0.1 g (0.004 oz) |
| Perfume | 0.15 g (0.005 oz) |
| Water | balance to 100 g (3.53 oz) |

The composition is charged into a container with a mechanically driven foam pump device.

### Example 14: pump setting foam

| | |
|---|---|
| Polymer no. 1 or 16A or 16B | 27 g (0.10 oz) |
| Polyquaternium-6 | 0.35 g (0.012 oz) |
| Cocamidopropyl hydroxysultaine | 0.4 g (0.01 oz) |
| Panthenol | 0.1 g (0.004 oz) |
| Ethanol | 8.9 g (0.31 oz) |
| Betaine | 0.1 g (0.004 oz) |
| Perfume | 0.15 g (0.005 oz) |
| Water | balance to 100 g (3.53 oz) |

The composition is charged into a container with a mechanically driven foam pump device.

### Example 15: pump setting foam

| | |
|---|---|
| Polymer no. 1 or 16A or 16B | 2.5 g (0.09 oz) |
| Ethanol | 8.9 g (0.31 oz) |
| Cocamidopropyl hydroxysultaine | 0.2 g (0.007 oz) |
| Cetyltrimethylammonium chloride | 0.2 g (0.007 oz) |
| Perfume | 0.15 g (0.005 oz) |
| Silk fibroin hydrolysate (Silkpro®) | 0.1 g (0.004 oz) |
| Water | balance to 100 g (3.53 oz) |

The composition is charged into a container with a mechanically driven foam pump device.

### Example 16: pump setting foam

| | |
|---|---|
| Polymer no. 5 or 16A or 16B | 2.0 g (0.07 oz) |
| Polyquaternium-4 (Celquat® L200) | 0.3 g (0.01 oz) |
| Ethanol | 8.9g (0.31 oz) |
| Cocamidopropyl hydroxysultaine | 0.2 g (0.007 oz) |
| Cetyltrimethylammonium chloride | 0.2 g (0.007 oz) |
| Perfume | 0.15 g (0.005 oz) |
| Citric acid | 0.1 g (0.004 oz) |
| Betaine | 0.1 g (0.004 oz) |
| Water | balance to 100 g (3.53 oz) |

The composition is charged into a container with a mechanically driven foam pump device.

### Example 17: pump setting foam

| | |
|---|---|
| Polymer no. 12 or 16A or 16B | 3.3 g (0.12 oz) |
| Polyquaternium-11 | 0.3 g (0.01 oz) |
| Cocamidopropyl hydroxysultaine | 0.4 g (0.01 oz) |
| Propylene glycol | 1.0 g (0.04 oz) |
| Methylparaben | 0.2 g (0.007 oz) |
| Perfume | 0.15 g (0.005 oz) |
| Water | balance to 100 g (3.53 oz) |

The composition is charged into a container with a mechanically driven foam pump device.

### Example 18: pump setting foam

| | |
|---|---|
| Polymer no. 2 or 16A or 16B | 2.8 g (0.10 oz) |
| Cocamidopropyl hydroxysultaine | 0.4 g (0.01 oz) |
| Rosmary leaf extract (Extrapon® Rosmarin) | 0.1 g (0.004 oz) |
| Ethanol | 8.9 g (0.31 oz) |
| Extrapon® seven herb plant extract | 0.1 g (0.004 oz) |
| Panthenyl ethyl ether | 0.1 g (0.004 oz) |
| Perfume | 0.15 g (0.005 oz) |
| Water | balance to 100 g (3.53 oz) |

The composition is charged into a container with a mechanically driven foam pump device.

### Example 19: aerosol setting foam - normal hold

| | |
|---|---|
| Polymer no. 2 or 16A or 16B | 3.5 g (0.12 oz) |
| Monobutyl ester of methyl vinyl ether/ maleic acid copolymer | 0.5 g (0.02 oz) |
| Butane | 4.0 g (0.14 oz) |
| Propane | 4.0 g (0.14 oz) |
| Ethanol | 8.9 g (0.31 oz) |
| PEG-25 PABA | 0.4 g (0.01 oz) |
| Betaine | 0.15 g (0.005 oz) |
| Perfume | 0.15g (0.005 oz) |
| Laureth-4 | 0.2 g (0.007 oz) |
| Cetrimonium bromide | 0.05 g (0.002 oz) |
| Amodimethicone | 0.5 g (0.02 oz) |
| Water | balance to 100 g (3.53 oz) |

The composition is charged into an aerosol can with a foam head.

### Examples 20: aerosol setting foam - normal hold

| | |
|---|---|
| Polymer no. 3 or 16A or 16B | 2.5 g (0.09 oz) |
| Polyquaternium-47 | 0.5 g (0.02 oz) |
| Butane | 4.0 g (0.14 oz) |
| Propane | 4.0 g (0.14 oz) |
| Betaine | 0.15 g (0.005 oz) |
| Dow Corning 1401 ¹⁾ | 0.25 g (0.009 oz) |
| 2-Ethylhexyl 4-methoxycinnamate | 0.2 g (0.07 oz) |
| Perfume | 0.15 g (0.005 oz) |
| Laureth-4 | 0.2 g (0.007 oz) |
| Cetrimonium chloride | 0.07 g (0.002 oz) |
| Water | balance to 100 g (3.53 oz) |

| | |
|---|---|
| ¹⁾ Dimethiconol, 13% in cyclomethicone | |

The composition is charged into an aerosol can with a foam head.

### Example21: aerosol setting foam - extra strong hold

| | |
|---|---|
| Polymer no. 5 or 16A or 16B | 4.3 g (0.15 oz) |
| Copolymer 845 (ISP)¹⁾ | 2.5 g (0.09 oz) |
| Polyquaternium-4 | 1.0 g (0.04 oz) |
| Butane | 4.0 g (0.14 oz) |
| Propane | 4.0 g (0.14oz) |
| Panthenol | 0.2 g (0.007 oz) |
| Perfume | 0.2 g (0.007 oz) |
| Abilquat® 3270 ²⁾ | 0.7 g (0.02 oz) |
| Cetrimonium chloride | 0.07 g (0.002 oz) |
| Water | balance to 100 g (3.53 oz) I |

| | |
|---|---|
| ¹⁾ VP/dimethylaminoethylmethacrylate copolymer, 20% in water ²⁾ Quaternium-80, 50% in propylene glycol | |

The composition is charged into an aerosol can with a foam head.

### Example 22: aerosol setting foam: - extra strong hold

| | |
|---|---|
| Polymer no. 1 or 16A or 16B | 3.9 g (0.14 oz) |
| Vinyl acetate/crotonic acid copolymer | 0.6 g (0.02 oz) |
| Polyquaternium-7 | 0.5 g (0.02 oz) |
| Butane | 4.0 g (0.14 oz) |
| Propane | 4.0 g (0.14 oz) |
| Ethanol | 8.9 g (0.31 oz) |
| PEG-25 PABA | 0.4 g (0.14 oz) |
| Panthenol | 0.2 g (0.007 oz) |
| Perfume | 0.2 g (0.007 oz) |
| Laureth-4 | 0.2 g (0.007 oz) |
| C9-11 Pareth-8 | 0.07 g (0.002oz) |
| Water | balance to 100 g (3.53 oz) |

The composition is charged into an aerosol can with a foam head.

### Example 23 spray setting lotion

| | |
|---|---|
| Polymer no. 1 or 16A or 16B | 2.7 g (0.10 oz) |
| Aquaflex® FX-64 ¹⁾ | 1.0 g (0.04 oz) |
| Ethanol | 2.7 g (0.10 oz) |
| Polyquaternium-35 | 1.0 g (0.04 oz) |
| PEG-25 PABA | 0.7 g (0.02 oz) |
| Panthenol | 0.35 g (0.012 oz) |
| Perfume | 0.25 g (0.009 oz) |
| Cetrimonium chloride | 0.2 g (0.007 oz) |
| PEG-40 hydrogenated castor oil | 0.21 g (0.007oz) |
| Water | balance to 100 g (3.53 oz) |

| | |
|---|---|
| ¹⁾ Isobutylene/etbylmaleimide/hydroxyethylmaleimide copolymer, 40% in water/ethanol The composition is charged into a container with spray pump device. | |

### Example 24: spray setting lotion

| | |
|---|---|
| Polymer no. 4 or 16A or 16B | 3.5 g (0.12 oz) |
| Octylacrylamide/acrylate/butylaminoethyl methacrylate copolymer (Amphomer®) | 2.0 g (0.07 oz) |
| Ethanol | 28.5 g (1.01 oz) |
| Aminomethylpropanol 95% | 0.6 g (0.02 oz) |
| Perfume | 0.25 g (0.009oz) |
| Cetyltrimethylammonium bromide | 0.20 g (0.007 oz) |
| Water | balance to 60 g (2.1 oz) |

The composition is charged into a container with spray pump device.

### Example25: spray setting lotion

| | |
|---|---|
| Polymer no. 13 or 16A or 16B | 1.0 g (0.04 oz) |
| Octylacrylamide/acrylates/butylaminoethyl methacrylate copolymer (Amphomer®) | 0.65 g (0.023 oz) |
| Polyquaternium-4 (Celquat® L200) | 0.2 g (0.007 oz) |
| Ethanol | 28.5 g (1.01 oz) |
| Aminomethylpropanol 95% | 0.6 g (0.02 oz) |
| Perfume | 0.25 g (0.009 oz) |
| Cetyltrimethylammonium chloride | 0.20 g (0.007 oz) |
| Water | balance to 60 g (2.1 oz) |

The composition is charged into a container with spray pump device.

### Example 26: Non-aerosol blow dry lotion

| | |
|---|---|
| Polymer no. 1 or 16A or 16B | 4.8 g (0.17 oz) |
| Vinyl caprolactam/VP/dimethylaminoethyl methacrylate copolymer (Advantage® S) | 2.0 g (0.07 oz) |
| Ethanol | 28.5 g (1.01 oz) |
| Perfume | 0.25 g (0.009 oz) |
| Cetyltrimethylammonium chloride | 0.20 g (0.007 oz) |
| Water | balance to 60 g (2.1 oz) |

### Example 27: non-aerosol blow dry lotion

| | |
|---|---|
| Polymer no. 7 or 16A or 16B | 3.1 g (0.11 oz) |
| Polyquaternium-4 (Celquat® L200) | 0.05 g (0.002 oz) |
| Diaformer® Z-71 1 ¹⁾ | 0.5 g (0.02 oz) |
| Ethanol | 27 g (0.95 oz) |
| Betaine | 0.1 g (0.004 oz) |
| Perfume | 0.25 g (0.009 oz) |
| PEG-40 hydrogenated castor oil | 0.21 g (0.007 oz) |
| Cetyltrimethylammonium bromide | 0.20 g (0.007 oz) |
| Water | balance to 100 g (3.53 oz) |

| | |
|---|---|
| ¹⁾ Acrylates/lauryl acrylate/stearyl acrylate/ethylamine oxide methacrylate copolymer | |

### Example 28: aerosol blow dry lotion

| | |
|---|---|
| Polymer no. 14 or 16A or 16B | 3.00 g (0.106 oz) |
| Sodium polystyrene sulfonate (Flexan®) | 2.30 g (0.081 oz) |
| Perfume | 0.20 g (0.007 oz) |
| Phenyltrimethicon (Baysilon® oil PD 5) | 0.02 g (0.001 oz) |
| Water | 10.00 g (0.3527 oz) |
| Ethanol | balance to 100 g (3.53 oz) |

The active substance solution is charged into an aerosol can with DME as propellant in a ratio of 45:55.

### Example 29: VOC 80 pump spray - strong hold

| | |
|---|---|
| Polymer no. 10 or 16A or 16B | 6.5 g (0.23 oz) |
| t-Butyl acryate/ethyl acrylate/methacrylic acid copolymer (Luvimer® 100 P) | 0.5 g (0.02 oz) |
| Perfume | 0.2 g (0.007 oz) |
| AMP | 0.10 g (0.004 oz) |
| Betaine | 0.05 g (0.002 oz) |
| Ethanol | 55 g (1.9 oz) |
| Water | balance to 100 g (3.53 oz) |

The composition is charged into a container with a spray pump device.

### Example 30: aerosol hair spray

| | |
|---|---|
| Octyl acrylamide/acrylic acid/butylaminoethyl methacrylate/methyl methacrylate/hydroxypropyl methacrylate copolymer (Amphomer®) | 3.00 g (0.106 oz) |
| Polymer no. 11 or 16A or 16B | 2.75 g (0.097 oz) |
| Phenyltrimethicone (Baysilon® oil PD 5) | 0.02 g (0.001 oz) |
| Perfume | 0.20 g (0.007 oz) |
| Water | 10.00g(0.3527oz) |
| AMP-95 | 0.48 g (0.017 oz) |
| Ethanol 510 | balance to 100 g (3.53 oz) |

The active substance solution is charged into an aerosol can with DME as propellant in a ratio of 45:55.

### Example31: aerosol hair spray

| | |
|---|---|
| t-Butyl acrylate/ethyl acrylate/methacrylic acid copolymer (Luvimer® 100 P) | 3.3 g (0.12 oz) |
| Polymer no. 13 or 16A or 16B | 3.3 g (0.12 oz) |
| VA/CROTONATES COPOLYMER (Luviset® CA 66) | 1.0 g (0.04 oz) |
| Perfume | 0.2 g (0.007 oz) |
| Water | 10.0 g (0.353 oz) |
| AMP-95 | 0.84 g (0.030 oz) |
| Ethanol 510 | balance to 100 g (3.53 oz) |

The active substance solution is charged into an aerosol can with DME as propellant in a ratio of 45:55.

### Example 32: aerosol hair spray

| | |
|---|---|
| Polymer no. 8 or 16A or 16B | 3.80 g (0.134 oz) |
| t-Butyl acrylate/ethyl acrylate/methacrylic acid copolymer (Luvimer® 100 P) | 3.30 g (0.126 oz) |
| Aminomethylpropanol 95% | 0.85g (0.030 oz) |
| Perfume | 0.20 g (0.007 oz) |
| Phenyltrimethicone (Baysilon® Öl PD 5) | 0.02 g (0.001 oz) |
| Water | 10.00 g (0.3527 oz) |
| Ethanol | balance to 100 g (3.53 oz) |

The active substance solution is charged into an aerosol can with DME as propellant in a ratio of 45:55.

## Claims

1. Use of a fluorescent polymer for the preparation of a cosmetic composition for the treatment of human hair, whereby the composition is selected from hair waxes, hair conditioners, hair lotions, hair gels, hair creams, hair foams, hair sprays and shampoos, wherein the fluorescent polymer is constructed from at least one radically polymerizable, unsaturated ethylenic fluorescent type of monomer, which is selected from monomers of formulae 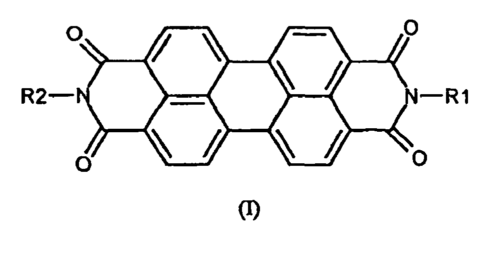 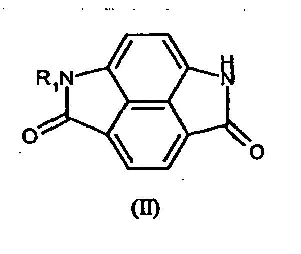 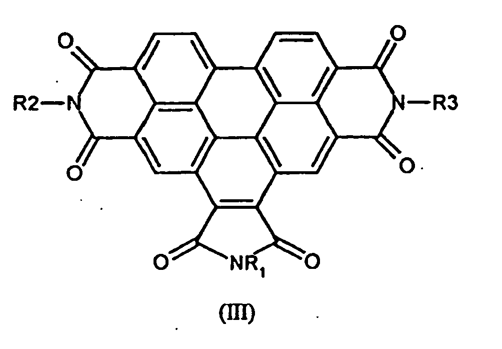 in which R1 stands for an unsaturated ethylenic group and R2 and R3 independently from one another stand for hydrogen or a linear or branched C1-24 alkyl group, or for a residue of the formula (IV) or (V)

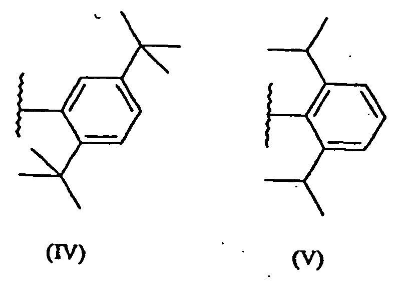

2. Use as described in claim 1, **characterized in that** the residue R1 has the general formula -X-CR=CH2, in which X stands for a C1 to C18 alkylene group or a phenylene group and R stands for hydrogen or a linear or branched C1-24 alkyl group, or for a residue of the formula (IV) or (V)

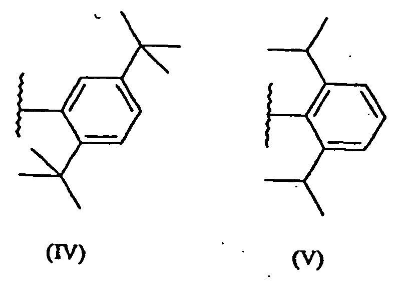

3. Use as described in one of the previous claims, **characterized in that** the fluorescent polymer is a copolymer from at least one first, radically polymerizable, unsaturated ethylenic fluorescent type of monomer and one or more radically polymerizable, unsaturated ethylenic non-fluorescent comonomers.

4. Use as described in the previous claim, **characterized in that** the comonomers are selected from
- acrylamide, methacrylamide, alkyl- and dialkylacrylamide, alkyl and dialkylmethacrylamide, alkyl acrylate, alkyl methacrylate, vinylcaprolactone, vinylcaprolactam, vinylpyrrolidone, vinyl esters, vinyl ethers, vinyl alcohol, styrenes and alkyl- or aryl-substituted cyclic maleimides;
- aminoalkyl acrylate, aminoalkyl methacrylate, dialkylaminoalkyl acrylate, dialkylaminoalkyl methacrylate, monoalkylamino alkylacrylate and monoalkylaminoalkyl methacrylate, dialkylaminoalkyl acrylamide, dialkylaminoalkyl methacrylamide;
- acrylic acid, methacrylic acid, crotonic acid, maleic anhydride, maleic acid monoesters, itaconic acid, styrene sulfonate, amidoalkylacrylamide sulfonate;
- alkylaminoxide methacrylate, methacryloylalkylbetaine, methacrylamidoalkyltrialkylammonium, acrylamidoalkyltrialkylammonium, quaternary crotonbetaines, quaternary crotonbetaine esters, trialkylammoniumalkyl methacrylate, trialkylammoniumalkyl acrylate, dialkyldiallylammonium, alkylvinylimidazolium, alkylvinylpyridinium.

5. Polymer constructed of at least one fluorescent type of monomer that contains at least one naphthalene bislactam structural unit or at least one perylene hexacarboxylic acid trisimide structural unit, wherein the fluorescent type of monomer is selected from monomers of the structures (II) and (III) according to claim 1.

6. Polymer as described in claim 5, c**characterized in that** it is constructed of at least one monomer selected from 1-alkenyl-1,6-dihydroisoindolo[6,7,1-*cde*]indole-2,5-dione and N¹-alkenyl-[N²,N³-bis (alkyl)-benzo[ghi]perylene-2,3,8,9,11, 12-hexacarboxylic acid-2,3:8,9:11,12-tris(dicarboximide). dialkyldiallylammonium, alkylvinylimidazolium, alkylvinylpyridinium.

7. Radically polymerizable, unsaturated ethylenic fluorescent compound selected from vinyl-substituted naphthalene bislactams and vinyl-substituted perylene hexacarboxylic acid trisimides, wherein the compound has a structure according to formula (II) or formula (III) of claim 1.

8. Compound according to claim 7, **characterized in that it** is selected from 1-alkenyl-1,6-dihydroisoindolo[6,7,1-*cde*]indole-2,5-diones and N'-alkenyl-[N²,N³-bis(alkyl)-benzo[ghi]perylene-2,3,8,9,11,12-hexacarboxylic acid-2,3:8,9:11,12-tris(dicarboximides).

## Patentansprüche

1. Verwendung eines fluoreszierenden Polymers zur Herstellung einer Kosmetikzusammensetzung zur Behandlung von menschlichem Haar, wobei die Zusammensetzung aus Haarwachsen, Haarspülungen, Haarlotionen, Haargelen, Haarcremes, Haarschäumen, Haarsprays und Shampoos ausgewählt ist, wobei das fluoreszierende Polymer aus mindestens einem radikalisch polymerisierbaren, ungesättigten ethylenischen fluoreszierenden Monomertyp aufgebaut ist, der aus Monomeren der folgenden Formeln ausgewählt ist 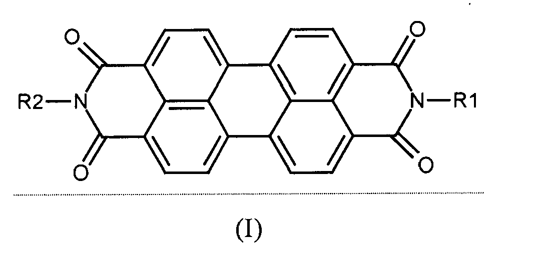 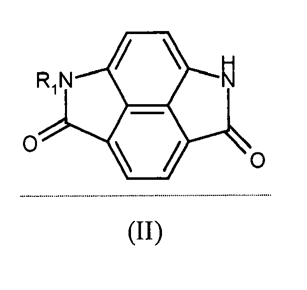 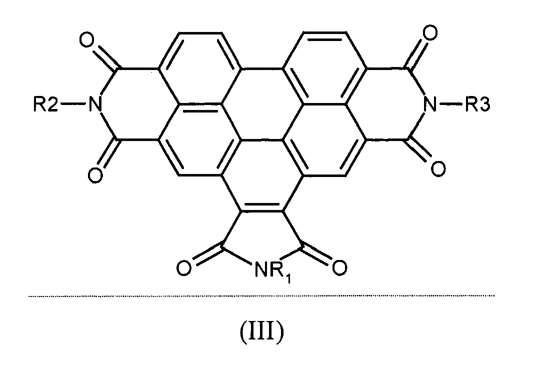 worin R1 für eine ungesättigte ethylenische Gruppe steht und R2 und R3 unabhängig voneinander für Wasserstoff oder eine lineare oder verzweigte C1-24-Alkylgruppe oder für einen Rest der Formel (IV) oder (V) stehen

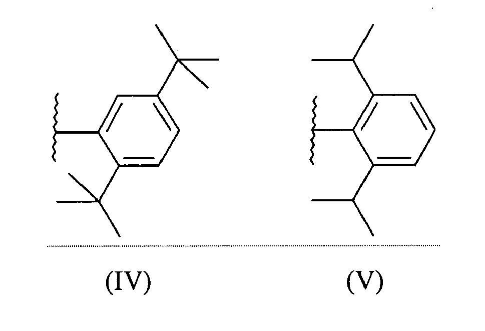

2. Verwendung wie in Anspruch 1 beschrieben, **dadurch gekennzeichnet, dass** der Rest R1 die allgemeine Formel -X-CR=CH2 hat, worin X für eine C1- bis C 18-Alkylengruppe oder eine Phenylengruppe steht und R für Wasserstoff oder eine lineare oder verzweigte C1-24-Alkylgruppe oder für einen Rest der Formel (IV) oder (V) steht

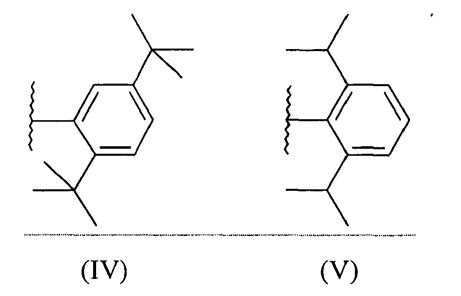

3. Verwendung wie in einem der vorstehenden Ansprüche beschrieben, **dadurch gekennzeichnet, dass** das fluoreszierende Polymer ein Copolymer aus mindestens einem ersten, radikalisch polymerisierbaren, ungesättigten ethylenischen fluoreszierenden Monomertyp und einem oder mehreren radikalisch polymerisierbaren, ungesättigten ethylenischen nichtfluoreszierenden Comonomeren ist.

4. Verwendung wie im vorstehenden Anspruch beschrieben, **dadurch gekennzeichnet, dass** die Comonomere aus folgenden ausgewählt sind
- Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylestern, Vinylethern, Vinylalkohol, Styrolen und alkyl- oder arylsubstituierten cyclischen Maleimiden;
- Aminoalkylacrylat, Aminoalkylmethacrylat, Dialkylaminoalkylacrylat, Dialkylaminoalkylmethacrylat, Monoalkylaminoalkylacrylat, Monoalkylaminoalkylmethacrylat, Dialkylaminoalkylacrylamid, Dialkylaminoalkylmethacrylamid;
- Acrylsäure, Methacrylsäure, Krotonsäure, Maleinsäureanhydrid, Maleinsäuremonoestern, Itaconsäure, Styrolsulfonat, Amidoalkylacrylamidsulfonat;
- Alkylaminoxidmethacrylat, Methacryloylalkylbetain, Methacrylamidoalkyltrialkylammonium, Acrylamidoalkyltrialkylammonium, quartäre Krotonbetaine, quartäre Krotonbetainester, Trialkylammoniumalkylmethacrylat, Trialkylammoniumalkylacrylat, Dialkyldiallylammonium, Alkylvinylimidazolium, Alkylvinylpyridinium.

5. Polymer, das aus mindestens einem fluoreszierenden Monomertyp aufgebaut ist, der mindestens eine strukturelle Naphthalinbislactam-Einheit oder mindestens eine strukturelle Perylenhexacarbonsäuretrisimid-Einheit enthält, wobei der fluoreszierende Monomertyp aus Monomeren der Strukturen (II) und (III) nach Anspruch 1 ausgewählt sind.

6. Polymer wie in Anspruch 5 beschrieben, **dadurch gekennzeichnet, dass** es aus mindestens einem Monomer, das aus 1-Alkenyl-1,6-dihydroisoindolo[6,7,1-*cde*]indol-2,5-dion, N'-Alkenyl-[N²,N³-bis(alkyl)-benzo[ghi]perylen-2,3,8,9,11, 12-hexacarbonsäure-2,3:8,9:11,12-tris(dicarboximid) ausgewählt ist, aufgebaut ist.

7. Radikalisch polymerisierbare, ungesättigte ethylenische fluoreszierende Verbindung, die aus vinylsubstituierten Naphthalinbislactamen und vinylsubstituierten Perylenhexacarbonsäuretrisimiden ausgewählt ist, wobei die Verbindung eine Struktur gemäß Formel (II) oder Formel (III) von Anspruch 1 hat.

8. Verbindung nach Anspruch 7, **dadurch gekennzeichnet, dass** sie aus 1-Alkenyl-1,6-Dihydroisoindolo[6,7,1-cde]indol-2,5-dionen und N¹-Alkenyl-[N²,N³-bis(alkyl)-benzo[ghi]perylen-2,3,8,9,11,12-hexacarbonsäure-2,3:8,9:11,12-tris-(dicarboximiden) ausgewählt ist.

## Revendications

1. Utilisation d'un polymère fluorescent pour la préparation d'une composition cosmétique pour le traitement de cheveux humains, selon quoi la composition est choisie parmi les cires capillaires, après-shampooings, lotions capillaires, gels pour cheveux, crèmes pour cheveux, mousses pour cheveux, aérosols pour cheveux et shampooings, dans laquelle le polymère fluorescent est construit à partir d'au moins un type de monomère fluorescent éthylénique insaturé radicalement polymérisable, qui est choisi parmi les monomères de formules 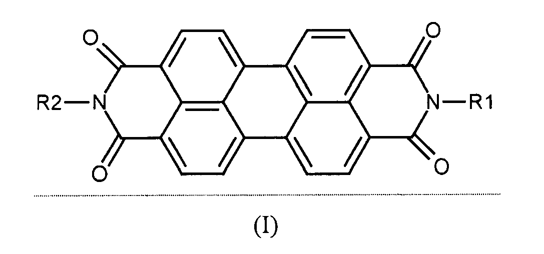 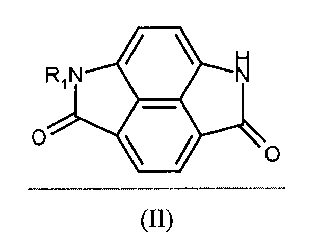 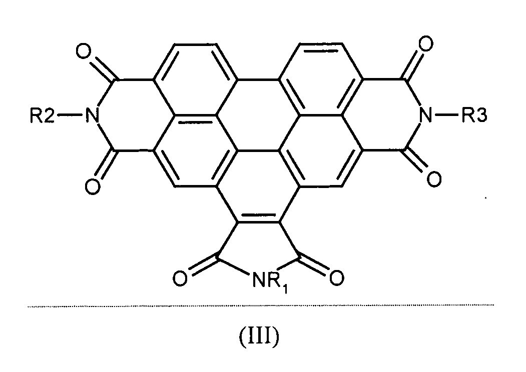 dans lesquelles R1 représente un groupe éthylénique insaturé et R2 et R3 indépendamment l'un de l'autre représentent un hydrogène ou un groupe alkyle linéaire ou ramifié en C1 à 24, ou un résidu de formule (IV) ou (V)

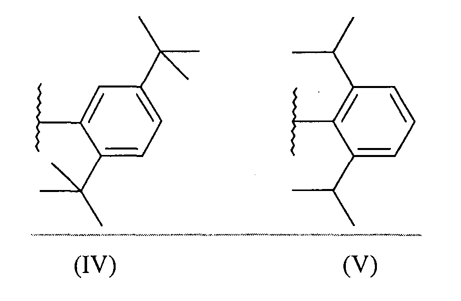

2. Utilisation selon la revendication 1, **caractérisée en ce que** le résidu R1 est de formule générale -X-CR=CH2, dans laquelle X représente un groupe alkylène en C1 à C18 ou un groupe phénylène et R représente un hydrogène ou un groupe alkyle linéaire ou ramifié en C1 à 24, ou un résidu de formule (IV) ou (V)

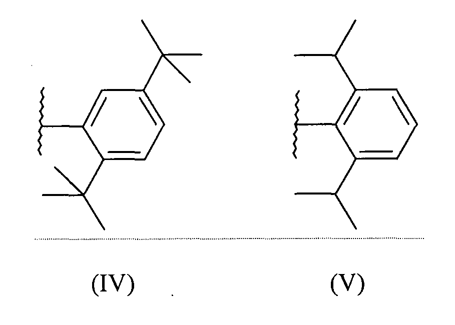

3. Utilisation telle que décrite dans une des revendications précédentes, **caractérisée en ce que** le polymère fluorescent est un copolymère provenant d'au moins un premier type de monomère fluorescent éthylénique insaturé radicalement polymérisable et un ou plusieurs comonomères non fluorescents éthyléniques insaturés radicalement polymérisables.

4. Utilisation telle que décrite dans la revendication précédente, **caractérisée en ce que** les comonomères sont choisis parmi
- acrylamide, méthacrylamide, alkyl- et dialkylacrylamide, alkyle et dialkylméthacrylamide, acrylate d'alkyle, méthacrylate d'alkyle, vinylcaprolactone, vinylcaprolactame, vinylpyrrolidone, esters de vinyle, vinyl-éthers, alcools vinyliques, styrènes et maléimides cycliques à substitution alkyle ou aryle ;
- acrylate d'aminoalkyle, méthacrylate d'aminoalkyle, acrylate de dialkylaminoalkyle, méthacrylate de dialkylaminoalkyle, monoalkylamino alkylacrylate, méthacrylate de monoalkylaminoalkyle, acrylamide de dialkylaminoalkyle, méthacrylamide de dialkylaminoalkyle ;
- acide acrylique, acide méthacrylique, acide crotonique, anhydride maléique, monoesters d'acide maléique, acide itaconique, sulfonate de styrène, sulfonate d'amido-alkylacrylamide ;
- méthacrylate d'alkylaminoxyde, méthacryloylalkylbétaïne, méthacrylamidoalkyltrialkylammonium, acrylamido-alkyltrialkylammonium, crotonbétaïnes quaternaires, esters de crotonbétaïne quaternaires, méthacrylate de trialkylammoniumalkyle, acrylate de trialkylammoniumalkyle, dialkyldiallylammonium, alkylvinylimidazolium, alkylvinylpyridinium.

5. Polymère construit d'au moins un type fluorescent de monomère qui contient au moins un motif structural naphtalène bislactam ou au moins un motif structural pérylène acide hexacarboxylique trisimide, où le type fluorescent de monomère est choisi parmi les monomères de structures (II) et (III) selon la revendication 1.

6. Polymère selon la revendication 5, **caractérisé en ce qu'**il est construit d'au moins un monomère choisi parmi 1-alcényl-1,6-dihydro-iso-indolo[6,7,1-*cde*]indole-2,5-dione, N¹-alcényl-[N²,N³-bis(alkyl)-benzo[ghi]pérylène-2,3,8,9,11, 12-hexacarboxylique acide-2,3:8,9:11,12-tris(dicarboximide).

7. Composé fluorescent éthylénique insaturé radicalement polymérisable choisi parmi les naphtalène bislactams à substitution vinyle et pérylène acide hexacarboxylique trisimides à substitution vinyle, où le composé a une structure selon la formule (II) ou la formule (III) de la revendication 1.

8. Composé selon la revendication 7, **caractérisé en ce qu'**il est choisi parmi les 1-alcényl-1,6-dihydro-iso-indolo[6,7,1-*cde*]indole-2,5-diones et N¹-alcényl-[N²,N³-bis(alkyl)-benzo[ghi]pérylène-2,3,8,9,11,12-acide hexacarboxylique-2,3:8,9:11,12-tris(dicarboximides).
